# EUROPEAN PATENT SPECIFICATION

(11) **EP 2 835 062 B1**
(45) Date of publication and mention of the grant of the patent: **05.09.2018**
(21) Application number: 12873614.7
(22) Date of filing: 14.12.2012
(51) Int. Cl.: A24F 47/00, A61M 15/06, A61M 11/04

(54) **ATOMIZATION DEVICE AND ELECTRONIC CIGARETTE THEREOF**
ZERSTÄUBUNGSVORRICHTUNG UND ELEKTRONISCHE ZIGARETTE DAMIT
DISPOSITIF D'ATOMISATION ET SA CIGARETTE ÉLECTRONIQUE

(30) Priority: 01.04.2012 CN 201220141170 U; 05.06.2012 WO PCT/CN2012/076492; 05.06.2012 WO PCT/CN2012/076493
(43) Date of publication of application: 11.02.2015
(73) Proprietor: Huizhou Kimree Technology Co., Ltd. Shenzhen Branch, Shenzhen City, Guangdong 518040 (CN)
(72) Inventor: LIU, Qiuming, Huizhou, Guangdong 516000 (CN)
(74) Representative: Beetz & Partner mbB
(86) International application number: PCT/CN2012/086685
(87) International publication number: WO 2013/149484

(56) References cited:
- EP-A1- 1 609 376
- EP-A1- 2 614 732
- CN-A- 102 326 869
- CN-U- 201 467 998
- CN-Y- 201 072 979
- KR-A- 20110 006 928
- KR-B1- 101 081 481
- US-A- 4 819 665
- US-A1- 2011 303 231

## Description

### FIELD OF THE INVENTION

The present invention relates to atomizing devices, and more particularly pertains to atomizing devices of smoking simulators and electronic cigarette using the atomizing device.

### BACKGROUND OF THE INVENTION

Current atomizer used in electronic cigarette generally uses absorbent cotton fixed on an atomizing seat. A heating wire is located in the absorbent cotton to heat tobacco flavored liquid. The electronic line of the heating wire needs to be welded to corresponding electrode. The current atomizer used in electronic cigarette has at least the following problems: the sealing of the atomizing seat for tobacco flavored liquid is not tight or balanced; the assembly of the atomizing device and the absorbent cotton is inconvenient; the operation of the heating wire is complicated, which easily makes welding poor, thus makes the atomizer out of work; welding residue is harmful to people. The document US 2011/303231 A1 discloses an atomizing device according to the preamble of claim 1. The document EP 2 614 732 A1 discloses a further atomizing device.

### SUMMARY OF THE INVENTION

The invention is only limited by the claims. An object of the present invention to provide an atomizing device which has good sealing, high strength, and easily assembled.

Described is a disposable electronic cigarette, in which atomizing device has good sealing, high strength, and easily assembled.

Described is a reusable electronic cigarette, in which atomizing device has good sealing, high strength, and easily assembled.

To achieve the above object, the present invention provides an atomizing device comprising an outer sleeve, oil storage layer, heating assembly, atomizing seat and hard connecting assembly. The oil storage layer, the heating assembly and the atomizing seat are assembled in the outer sleeve. The atomizing seat is an elastic atomizing seat. The hard connecting assembly is inserted in the elastic atomizing seat to elastically squeeze and tensioningly engage with the elastic atomizing seat.

In a further embodiment, the elastic atomizing seat is adapted to the outer sleeve. The elastic atomizing seat is elastically expanded as a result of the connecting assembly being inserted in the elastic atomizing seat to be tightly sealed in the inner wall of the outer sleeve.

In a further embodiment, the heating assembly comprises heating element and electronic wires connected to two end of the heating element. The electronic wires respectively are tensioningly fixed in the elastic atomizing seat and electrically connect to the connecting assembly.

In a further embodiment, the connecting assembly comprises conductive connector and a top electrode. The conductive connector and the top electrode are insulatively engage with each other. The elastic atomizing seat defines slot. The conductive head is tensioningly inserted into the slot. The elastic atomizing seat defines air channel therein. The top electrode is inserted the air channel to tensioningly engage with the inner wall of the elastic atomizing seat.

In a further embodiment, the heating assembly comprises a heating element and electronic wires connected to two end of the heating element. One electronic line is tensioningly fixed between the elastic atomizing seat and the connector to electrically connect to the connector. The other electronic line is tensioningly fixed between the air channel wall of the elastic atomizing seat and the top electrode to electrically connect to the top electrode. The conductive connector is metallic.

In a further embodiment, the elastic atomizing seat comprises main body and axial extending portion formed on the center of the main body. The air channel of the atomizing seat runs through the main body and the axial extending portion. The main body forms an annular flange on an outer edge thereof. The outer diameter of the annular flange is adapted to an inner diameter of the outer sleeve, tightly latches the inner wall of the outer sleeve to achieve seal. The annular flange and the outer wall of the main body cooperatively restrict the slot.

In a further embodiment, the elastic atomizing seat forms a pair of line holes used for the electronic wires passing through. The line holes have thin neck. When the atomizing seat is squeezed, the thin neck elastically deforms to tightly clamp the electronic wires therein.

In a further embodiment, the line holes longitudinally pass through the main body of the elastic atomizing device. One line hole communicates with the air channel. One electronic line passes through a corresponding line hole and bypasses a bottom edge of the main body to stretch into the slot and is latched in the inner wall of the slot by the connector. The other electronic line passes through the other corresponding line hole to go back the inner wall of the air channel and is clamped by the top electrode.

In a further embodiment, the outer wall of the connector forms an annular flange adapted to the inner diameter of the outer sleeve. The connector forms a stepped hole therein. When the connector is inserted into the slot, the annular flange of the connector longitudinally resists the annular flange of the atomizing seat. The main body the atomizing seat is tensioningly latched in the stepped hole of the connector. The top electrode is latched the stepped hole of the connector via an insulating ring, and longitudinally extending and tensioningly latches in the inner wall of the air channel of the main body of the atomizing seat.

In a further embodiment, the atomizing device further comprises glass fiber tube. The glass fiber tube defines assembling hole to fix the heating assembly. The glass fiber tube may be double layer tube. An inner layer tube forms a supporting ear corresponding to the assembling hole to fix the heating assembly. The elastic atomizing seat is made of silicon.

In a further embodiment, the oil storage layer is a storage cotton packaging on the outer of the glass fiber tube.

In a further embodiment, the glass fiber tube packaged by a cloth layer, and then is packaged by the storage cotton.

Described is a disposable electronic cigarette comprising the above-mentioned atomizing device, and further comprising battery assembly. The positive and negative electrodes respectively electrically connect with the connecting assembly.

In a further embodiment, the battery assembly is assembled in the outer sleeve.

Described is a reusable electronic cigarette comprising the above-mentioned atomizing device, and further comprising a power source pole. The power source pole connects with the atomizing device. The positive and negative electrodes of the power source pole respectively electrically connect with the connecting assembly.

The atomizing device and the electronic cigarette of the present invention has the following advantage: the strength of the atomizing seat covered by the connector or other related elements is greatly enhanced to ensure assembly convenient; avoiding an user's hand hold inconvenient to package cotton and too soft of the simply using silicon glue; avoiding leaking liquid, plugging holes as results of easy deflection. The atomizing seats is squeezed and sealed by the connector, of which sealing is better and avoid too soft of the simply using silicon glue, and also avoid inconvenient to assembly when a simple sealing ring moves with the outer sleeve assembling in. After assembling the atomizing seat of the heating assembly and the connector together, hand only holds the connector to package cotton, in which efficiency and yield are greatly improved. The electronic wires are not required to weld, which avoids all poor phenomenon.

The components in the drawings are not necessarily drawn to scale, the emphasis instead being placed upon illustrating the principles of the present disclosure. Moreover, in the drawings, like reference numerals designate corresponding parts throughout several views, and all the views are schematic.

### BRIEF DESCRIPTION OF THE DRAWINGS

FIG. 1 is a perspective view of the atomizing device according to an embodiment of the present invention.
FIG. 2 is a sectional view of the atomizing device according to an embodiment of the present invention.
FIG. 3 is a perspective, exploded view of the atomizing device according to an embodiment of the present invention.
FIG. 4 is a perspective view of the atomizing seat according to an embodiment of the present invention.
FIG. 5 is a sectional view of the atomizing seat according to an embodiment of the present invention.
FIG. 6 is a perspective view of the connector according to an embodiment of the present invention.
FIG. 7 is a sectional view of the connector according to an embodiment of the present invention.
FIG. 8 is sectional view of the atomizing device in semi-finished state according to the embodiment of the present invention.
FIG. 9 is sectional view of the electronic cigarette.
FIG. 10 is sectional view of the electronic cigarette.

### DETAILED DESCRIPTION OF THE INVENTION

It is needed to state that embodiments and all element limitations in all embodiments may be combined in the case of no conflicts. The present invention will be described in detail in the following combining figures and embodiments.

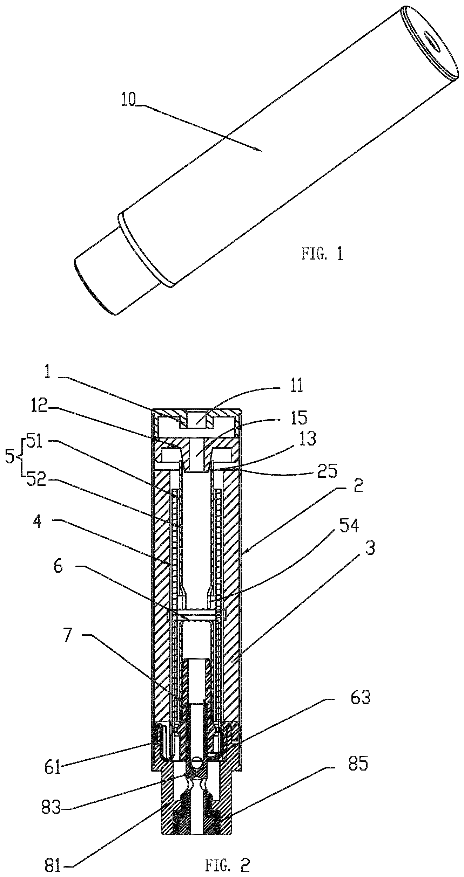

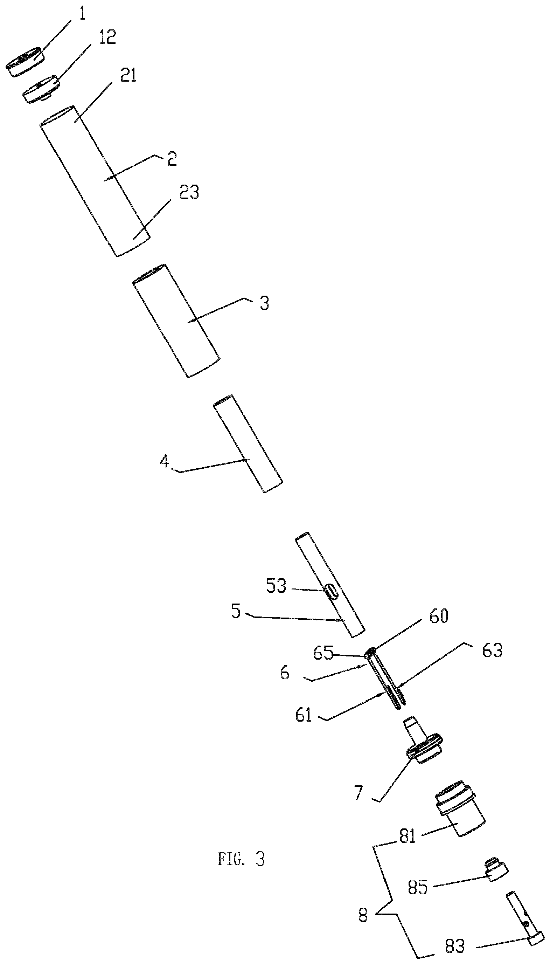

Referring to FIG. 1 to FIG. 3, an atomizing device 10 according to one embodiment of the present invention includes an inhalation element cover 1, an outer sleeve 2, an oil storage layer 3, a cloth layer 4, a glass fiber tube 5, a heating assembly 6, an atomizing seat 7, and a connecting assembly 8. The oil storage layer 3, the cloth layer 4, the glass fiber tube 5, and the heating assembly 6 are all assembled in the outer sleeve 2. The atomizing seat 7 and the connecting assembly 8 are reciprocally elastically squeezed and assembled at one end of the outer sleeve 2 or in the outer sleeve 2. The inhalation element cover 1 covers the other end of the outer sleeve 2. The atomizing seat 7 is an elastic atomizing seat, and defines slot 73 therein, and forms air channel 74 at a center thereof. The connecting assembly 8 includes a connector 81 and a top electrode 83. The connector 81 and the top electrode 83 is electrically insulated and assembled together. The connecting assembly 8 is inserted into the elastic atomizing seat 7 and elastically engages with the elastic atomizing seat 7 by squeezing. Concretely, the connector 81 is tensioningly inserted into the slot 73 of the elastic atomizing seat 7, and the top electrode 83 is tensioningly inserted into the air channel 74 of the atomizing seat 7. The atomizing seat has a higher strength after sleeved by the connecting assembly 8, which benefits to assembly of other element. The atomizing seat 7 can be better sealed at one end or inner wall of the outer sleeve 3.

The atomizing sleeve 2 is hollow tube-shaped in this embodiment. Two ends of the outer sleeve 2 are an inhalation element end 21 and a connecting end 23. The inhalation element cover 1 and the connecting assembly 8 are respectively assembled the inhalation element end 21 and the connecting end 23. The oil storage layer 3, the cloth layer 4, the glass fiber tube 5, and the heating assembly 6 are fixed in the outer sleeve 2. The atomizing seat 7 is fixed and sealed in the connecting end 23 of the outer sleeve 2 relative to the inhalation element cover 1, and also engages with the connecting assembly 8. The inhalation element end 21 of the outer sleeve 2 forms a baffle ring 25 at the inner wall thereof.

The inhalation element cover 1 is adapted to the outer sleeve 2. The inhalation element cover 1 is ring-shaped, and may be other shaped such as circular or taper-shaped. The material and shape of the inhalation element cover 1 is adaptive to user's mouth, such as made of soft material or woody material. The center of the inhalation element cover 1 defines an air hole 11. In this embodiment, to achieve a better sealing, the inhalation element cover 1 has a sealing ring 12 formed therein, which is adapted to an inner diameter of the outer sleeve 2. The inhalation element cover 1 and the sealing ring 12 seals the inhalation element end 21, and all defines air holes. The center of the sealing ring 12 or the inhalation element cover 1 longitudinally extends a supporting portion 13 (13') which is inserted into the glass fiber tube 5 to support one end of the glass fiber tube 5. The center of the sealing ring 12 defines an air hole 15 to communicate with the air hole 11 of the inhalation element cover 1 and the glass fiber tube 5. The edge of the sealing ring 12 resists the baffle ring 25 of the outer sleeve, and the supporting portion 23 faces to and inserts into the center hole of the baffle ring 25.

The oil storage layer 3 is a hollow tube in this embodiment and used to adsorb or storage tobacco flavored liquid, and located in the outer sleeve 2. Preferably, the oil storage layer 3 is made of material having the function of adsorb or storage such as cotton or fiber. The oil storage layer 3 is axially assembled in the inner wall of the outer sleeve 2, and two ends of the oil storage layer 3 respectively resist the baffle ring 25 of the outer sleeve 25 and the atomizing seat 7.

The embodiment further discloses the cloth layer 4 located between the glass fiber tube 5 and the oil storage layer 3. The cloth layer 4 may be made cotton cloth, fiber cloth, polymer cloth and so on. A main function of the cloth layer 4 is to package the glass fiber tube 5 to stably assemble the glass fiber tube 5, the heating assembly 6, the atomizing seat 7, and the connecting assembly 8 together to ensure electric circuit stably connected and reliable sealing. In addition, the function of the cloth layer 4 is to ensure the tobacco flavored liquid being conducted to the heating assembly 6 from the oil storage layer 3 to avoid leaking of the tobacco flavored liquid along between the outer sleeve 2 and the connector 81.

The glass fiber tube 5 is hollow tube and fixed in the cloth layer 4. Correspondingly, the oil storage layer 3 and the cloth layer 4 package on an outer of the glass fiber tube 5 to form tube. The tube wall of the glass fiber tube 5 defines an assembling hole 53 to assembly the heating assembly 6. The glass fiber tube 5 is longitudinally and axially supported in the oil storage layer 3 and the cloth layer 4. One end of the glass fiber tube 5 is latched between the baffle ring 25 of the outer sleeve 2 and the supporting portion 13 of the sealing ring 12 to fix the sealing ring 12 or the inhalation element cover 1 and the glass fiber tube 5. The other end of the glass fiber tube 5 is latched in the atomizing seat 7. Preferably, the inner wall of the assembling hole 53 forms a pair of supporting ears 54 to be beneficial to support the heating assembly 6. Furthermore, to firm the glass fiber tube 5, the oil storage layer 3 and the cloth layer 4, the glass fiber tube 5 is designed to be a double layer tube comprising an inner layer tube 51 and an outer layer tube 52. The outer layer tube 52 is further impactedly assembled on the heating assembly 6 of the inner layer tube 51. It is understood that the glass fiber tube 5 may be a single layer tube.

The heating assembly 6 includes a heating wire 60 and electronic wires 61,63 (respectively negative or positive electrode) extending from the two end of the heating wire 60. The heating wire 60 can be wrapped or folded, or other structure to contact with the oil storage layer 3 and the cloth layer 4, used to heat and vaporize the tobacco flavored liquid. In this embodiment, the heating wire 60 is wrapped to coil-shaped, in which is inserted a liquid conducting belt or a liquid conducting post 65 to fix the heating wire 60 in the glass fiber tube 5. Two ends of the liquid conducting belt (post) 65 radially pass through the assembling hole 53 of the glass fiber tube 5 and resist to the oil storage layer 3 so as to adsorb tobacco flavored liquid which is vaporized by the heating wire 60.

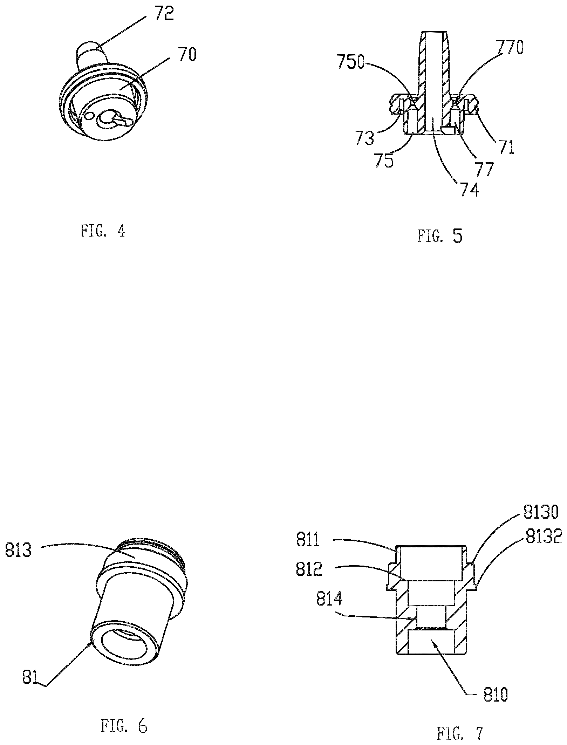

Referring also to FIG. 4 to FIG. 5, the elastic atomizing seat 7 is silicon atomizing seat, and includes a main body 70 and an extending portion 72. The main body 70 is substantially column-shaped. The extending portion 72 is columnar, and axially extends a predetermined distance from the center of the main body 70. After assembly, the extending portion 72 is tightly inserted into one end of the glass fiber tube 5 away from the inhalation element cover 1. The extending portion 72 and the main body 70 cooperatively defines the central air channel 74 which communicates with outer environment and the glass fiber tube 5, thus communicates with air hole 11 of the inhalation element cover 1 and the air hole 15 of the sealing ring 12, and lastly communicates with outer environment to form air channel. The main body 70 forms an annular flange 71 on an outer edge thereof. The outer diameter of the annular flange 71 is adapted to an inner diameter of the outer sleeve 2, and to tightly latch with the inner wall of the connecting end 23 of the outer sleeve 2. The annular flange 71 and the outer wall of the main body 70 cooperatively restrict the slot 73 which is an annular slot. The main body 70 defines a pair of line holes 75, 77. One line hole 77 communicates with the central air channel 74. Preferably, each line hole 75, 77 is disposed to have different inner diameter, and respectively has thin necks 750, 770 used for the electronic wires 61, 63 passing through and clamped by the thin neck 750, 770. In this embodiment, the main body 70 is cap-liked, and the annular flange 71 is same as to cap edge. Obviously, the main body 70 may be other shape.

Referring to FIG. 6 to FIG. 7, the connector 81 is a hard conductive element, and preferably metallic element. The top electrode 83 is inserted into the connector 81, and an insulating ring 85 is inserted between the top electrode 83 and the connector 81 to achieve insulate. The connector 81 is substantially hollow column and defines a stepped hole 810. The inner wall of the stepped hole 810 forms a first inner shoulder 812 and a second inner shoulder 814. One end of the connector 81 being inserted into the slot 73 of the atomizing seat 7 is an inserting end 811. Corresponding to a depth of the slot 73 of the atomizing seat 7 and the annular flange 71, the outer wall of the connector 81 spaced from the inserting end 811 a predetermined distance forms an outer annular flange 813. The inserting end 811 is inserted into the slot 73 of the atomizing seat 7 to support the elastic atomizing seat 7, so as to make the elastic atomizing seat 7 having higher strength. In one embodiment, the outer annular flange 813 may be designed to be stepped shape, and includes a first step 8130 and a second step 8132. The outer diameter of the first step 8130 is adapted to the inner diameter of the outer sleeve 2, and tightly latches in the connecting end 23 of the outer sleeve 2, and also longitudinally resists a distal end of the annular flange 71 of the atomizing seat 7. The main body 70 of the atomizing seat 7 is tensioningly inserted into the stepped hole 810 and resists the first inner shoulder 812 so as to further seal and fix the outer sleeve 2 and the atomizing seat 7. The edge of the connecting end 23 of the outer sleeve 2 resists on the second step 8132. The second inner shoulder 814 is in fact a protruding ring formed on the inner wall of the connector 81. The insulating ring 85 is inserted into the stepped hole 810 via another end contrary to the inserting end 811 and latches with the second inner shoulder 814. The top electrode 83 is inserted into and latches in the insulating ring 85. The top electrode 83 is long columnar, and forms a flange at a distal end thereof to latch in the insulating ring 85. The column portion of the top electrode 83 is inserted into and tensioningly fixed the central air channel 74 of the atomizing seat 7 via the connector 81 to further support and elastically squeeze the silicon atomizing seat 7. The side wall of the top electrode 82 and the connector 81 correspondingly defines air hole (not labeled), which communicates with the air channel cooperatively defined by the connecting assembly 8, the atomizing seat 7, the glass fiber tube 5, the sealing ring 12 and the inhalation element cover 1 such that the outer air is adsorbed into and adsorbed out with the smoke produced by the atomizing device 10.

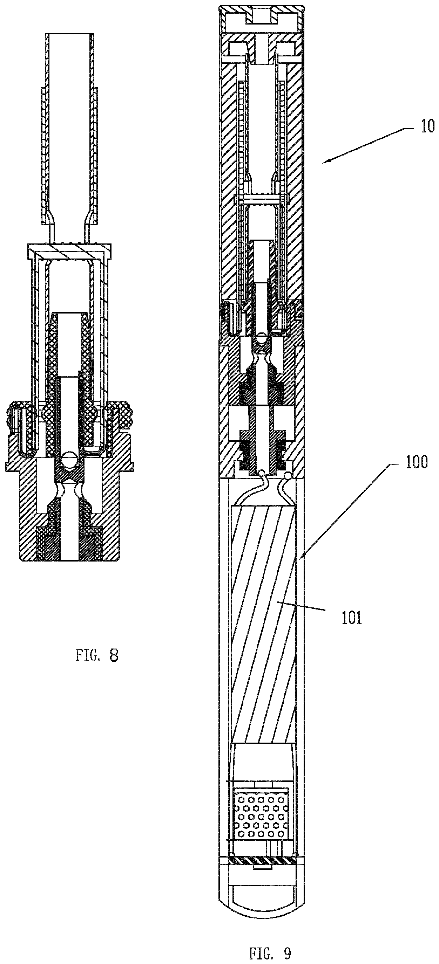

Referring to the FIG. 8 showing a semi-finished product, the heating wire 60 is fixed in the glass fiber tube 5, and the electronic wires 61, 63 pass throughout the assembling hole 50 and extend along the outer wall of the glass fiber tube 5. The two end of the liquid conducting belt (post) 65 passes throughout the assembling hole 50. At this time, the electronic wires 61, 63 respectively passes through the line holes 75, 77 of the silicon atomizing seat 7. The electronic wires 63 passes through the line hole 77 and bend into the inner wall of the central air channel 74 of the atomizing seat 7. The electronic line 61 passes through line hole 75 and then bends into the slot 73 from the edge of the main body 70. The inserting end 811 of the connector 81 is inserted into the slot 73 of the silicon atomizing seat 7 and tightly latches the electronic wires 61. The top electrode 83 is inserted in to the central air channel 74 of the atomizing seat 7 and tightly latches the electronic line 63. The column portion of the top electrode 83 squeezes the silicon atomizing seat 7 from the central air hole 74 of the atomizing seat 7. Therefore, the electronic wires 63 is pressed between the inner wall of the atomizing seat 7 and the outer wall of the top electrode 83, thus electrically connected with the top electrode 83. When the inserting end 811 of the metallic connector 81 is inserted into the slot 73 of the atomizing seat 7, the insert end 811 squeezes the annular flange 71 and the main body 70 of the atomizing seat 7 such that the electronic wires 63 is tightly latched in the slot 73 of the atomizing seat 7 and thus stably electrically connected with the metallic connector 81. After the metallic connector 81 and the top electrode 83 is inserted into the atomizing seat 7, the silicon atomizing seat 7 is elastically pressed inwards to further latch the electronic wires 61, 63 passing through the line holes 75, 77, especially latch in the thin necks 750,770. Therefore, the electronic wires 61, 63 cannot be pulled. Simultaneously, the annular flange 71 of the silicon atomizing seat 7 is pressed outwards and expands so as to evenly latch in the inner wall of the outer sleeve 2, thus achieve better sealing effect. Furthermore, after the connector 81 and the top electrode 83 are inserted into the silicon atomizing seat 7, the total hardness and strength of the atomizing device is enhanced. Thus, it is convenient to hold the connector 81 to package the cloth layer 4 and the oil storage layer 3 on the glass fiber tube 5. The oil storage layer 3 of the embodiment is made of packaging cotton. The assembly is convenient. After packaging the cloth layer 4 and the oil storage layer 3 on the glass fiber tube 5, the outer sleeve is packaged on the glass fiber tube 5. Then the inhalation element end 21 is inserted into the sealing ring 12 and the inhalation element cover 1. Thus the atomizing device 10 is completely assembled. With the aid of elastic engagement between the metallic connector 81, the top electrode 83 and the silicon atomizing seat 7, the electronic wires 61, 63 of the atomizing device 10 according to the present invention is tightly pressed in, and stably electrically connected with the metallic connector 81 and the top electrode 83, which needs no any welding, saves costs, and avoids producing any harmful matter. In addition, the silicon atomizing seat 7 is sleeved in the connecting assembly 8, and is pressed to expand outwards to make the atomizing seat 7 sealed in the outer sleeve 2 and does not deform and slide.

Referring to FIG. 9, the electronic cigarette includes the atomizing device 10 and a power source pole 100. The atomizing device 10 and the power source pole 100 are detachably or undetachably connected together. The power source pole 100 includes battery 101 disposed therein. One end of the power source pole 100 is adapted for a connecting end of the atomizing device 10, and may connect with the connecting end of the atomizing device 10 by thread connection, latching connection, tensioning connection, adhesive bonding, or integrally molding. In use, the power source pole 100 connects with the atomizing device 10, and the top electrode 83 and the metallic connector 81 are respectively connected with the positive and negative electrode of the power source pole 100 so as to supply power to the heating wire.

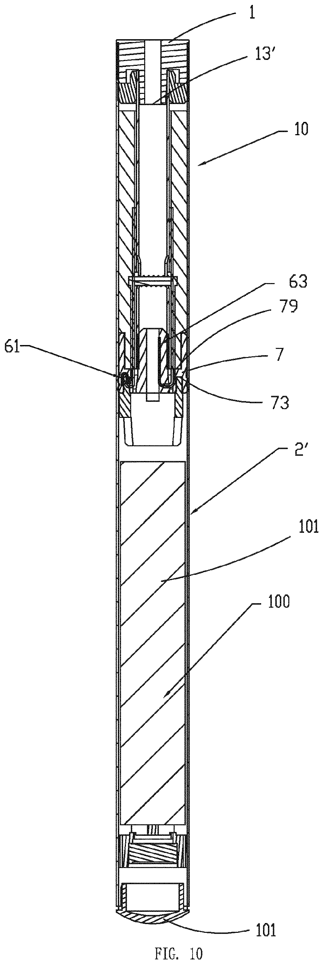

Referring to FIG. 10, a disposable electronic cigarette 100 is shown. The outer sleeve 2 comprises the continuous outer sleeve 2'of the electronic cigarette 100. The disposable electronic cigarette 100 is divided into two sections. One section is corresponding to the atomizing device 10, and the other section is corresponding to the power source pole 100. The assembling of each section is same as the above. The difference is that the atomizing seat 7 and the connecting assembly 8 are assembled in the outer sleeve 2', instead of being assembled in the distal end. The battery 101 is located in the outer sleeve 2'and engages with the connecting assembly 8. The positive and negative electrodes of a power supply source are respectively electrically connected with the connector and the electronic wires 63 of the heating assembly 6, thus to achieve electrical connection to the electronic wires 61, 63 of the heating assembly 6. The outer sleeve 2' has a battery cover 103 disposed at the other end thereof contrary to the inhalation element cover 1. In the disposable electronic cigarette 100, the positive and negative electrodes of the battery respectively electrically connected with the electronic lies 61, 63 of the heating assembly 6, thus the top electrode 83 may be omitted.

Referring also to FIG. 10, the inhalation element cover 1 and the sealing ring 12 are latched each other, and an extending portion 13' inserting into the glass fiber tube 5 may be designed to axially extend from the inhalation element cover 1. The silicon atomizing seat 7 may be designed to have H-shaped cross-section, thus form the slots 73, 79 at two ends thereof. The slot 79 is used to latch one inserting end of the glass fiber tube 5 so as to further fix and position.

It is understood that the electronic cigarette may be reusable electronic cigarette. The atomizing device 10 and the power source pole 100 may be undetachably connected together. The atomizing device 10 may have liquid cup to storage tobacco flavored liquid or other liquid matter. In other embodiment, tobacco flavored liquid may be repeatedly added. For example, the inhalation element cover 1 and the sealing ring 12 are configured to be removable structure to repeatedly pour into the tobacco flavored liquid.

The cloth layer 4 of the embodiment according to the present invention may be omitted according concrete requirements, or be replaced by other material. In addition, the heating wire 6 of the heating assembly 6 may be other heating element such as heating block, heating stick, or other heating ways. The electronic wires 61, 63 connected to the two ends of the heating element pass through and is tensioningly fixed in the elastic atomizing seat 7, and then electrically connects with the connecting assembly 8.

The above -mentioned is embodiments of the present invention. It is pointed out that various improvements and modifications can be made to the embodiments by those skilled in the art as defined by the appended claims.

## Claims

1. An atomizing device, comprising: an outer sleeve (2), an oil storage layer (3), a cloth layer (4), a heating assembly (6), an atomizing seat (7), a glass fiber tube (5) and a hard connecting assembly (8), wherein the glass fiber tube (5) is configured to be packaged by the oil storage layer (3) and the cloth layer (4) and to support the heating assembly (6), the oil storage layer (3), the heating assembly (6) and the atomizing seat (7) being assembled in the outer sleeve (2); **characterized in that** the atomizing seat (7) is an elastic atomizing seat and includes an extending portion (72) tightly inserted into one end of the glass fiber tube (5), the hard connecting assembly (8) is inserted in the elastic atomizing seat (7) to elastically squeeze and tensioningly engage with the elastic atomizing seat (7).

2. The atomizing device defined according to claim **1,** wherein the elastic atomizing seat (7) is adapted for the outer sleeve (2), and the elastic atomizing seat (7) is elastically expanded as a result of the connecting assembly (8) being inserted in the elastic atomizing seat (7) to be tightly sealed in the inner wall of the outer sleeve (2).

3. The atomizing device defined according to claim **1,** wherein the heating assembly (6) comprises a heating element (60) and electronic wires (61, 63) connected to two ends of the heating element (60), and the electronic wires (61, 63) respectively are tensioningly fixed in the elastic atomizing seat (7) and electrically connected to the connecting assembly (8).

4. The atomizing device defined according to claim **1,** wherein the connecting assembly (8) comprises a conductive connector (81) and a top electrode (83), wherein the conductive connector (81) and the top electrode (83) are engaged with each other with an insulating ring (85) inserted between the conductive connector (81) and a top electrode (83); the elastic atomizing seat defines an air channel (74) and a slot (73), and the conductive connector (81) is tensioningly inserted into the slot (73); the elastic atomizing seat (7) defines air channel therein, and the top electrode (83) is inserted into the air channel (74) to tensioningly engage with the inner wall of the elastic atomizing seat (7).

5. The atomizing device defined according to claim **4,** wherein the heating assembly comprises a heating element (60) and electronic wires (61, 63) connected to two ends of the heating element (60); one electronic line (61) is tensioningly fixed between the elastic atomizing seat (7) and the connector (81) to electrically connect to the connector (61); the other electronic line (63) is tensioningly fixed between the air channelwall of the elastic atomizing seat (7) and the top electrode (83) to electrically connect to the top electrode (83) and wherein the conductive connector (81) is metallic.

6. The atomizing device defined according to claim **5,** wherein the elastic atomizing seat (7) comprises main body (70) and an axially extending portion (72) formed on the center of the main body (70); the air channel (74) of the atomizing seat runs through the main body (70) and the axially extending portion (72); the main body (70) forms an annular flange (71) on an outer edge thereof; the outer diameter of the annular flange (71) is adapted to an inner diameter of the outer sleeve (2), and tightly latches the inner wall of the outer sleeve (2) to achieve a sealing effect; and the annular flange (71) and the outer wall of the main body (70) cooperatively restrict the slot (73).

7. The atomizing device defined according to claim **5,** wherein the elastic atomizing seat (7) forms a pair of line holes (75, 77) used for the electronic wires (61, 63) passing through; the line holes (75, 77) have a thin neck (750, 770), respectively, wherein the thin necks (750, 770) are configured such that when the atomizing seat (7) is squeezed, the thin neck (750, 770) elastically deforms to tightly clamp the electronic wires (61, 63) therein.

8. The atomizing device defined according to claim **7,** wherein the line holes (75, 77) longitudinally pass through the main body (70) of the elastic atomizing device (7), and one line hole (75, 77) communicates with the air channel (74); one electronic line (61) passes through a corresponding line hole (75) and bypasses a bottom edge of the main body (70) to stretch into the slot (73) and is latched in the inner wall of the slot (73) by the connector (81); the other electronic line (63) passes through the other corresponding line hole (77) to go back the inner wall of the air channel (74) and is clamped by the top electrode (83).

9. The atomizing device defined according to claim **6,** wherein the outer wall of the connector (81) forms an annular flange (813) adapted to the inner diameter of the outer sleeve (2), and the connector (81) forms a stepped hole therein; when the connector (81) is inserted into the slot (73), the annular flange (813) of the connector (81) longitudinally resists the annular flange (813) of the atomizing seat (7), and the main body (70) of the atomizing seat (7) is tensioningly latched in the stepped hole of the connector (81); the top electrode (83) is latched in the stepped hole of the connector (81) via an insulating ring (85), and longitudinally extends and tensioningly latches in the inner wall of the air channel (74) of the main body (70) of the atomizing seat (7).

10. The atomizing device defined according to claim 1, wherein the glass fiber tube (5) defines an assembling hole (53) to fix the heating assembly (6); the glass fiber tube (5) is a double layer tube including an inner layer tube (51); said inner layer tube (51) forms a supporting ear (54) corresponding to the assembling hole (53) to fix the heating assembly (6); and the elastic atomizing seat (7) is made of silicon.

11. The atomizing device defined according to claim **10,** wherein the oil storage layer (3) is storage cotton packaging the glass fiber tube.

12. The atomizing device defined according to claim **11,** wherein the glass fiber tube (5) is packaged by the cloth layer (4), and then is packaged by the storage cotton.

13. An electronic cigarette, comprising an atomizing device claimed in one of claims **1** to **12,** and further comprising a battery assembly (101), the positive and negative electrodes of a battery respectively being electrically connected with the connecting assembly (8).

14. The electronic cigarette defined according to claim **13,** wherein the battery assembly (101) is assembled in the outer sleeve (2).

15. An electronic cigarette comprising an atomizing device claimed in any one of claims **1** to **12,** and further comprising a power source pole, the power source pole connecting with the atomizing device (7), the positive and negative electrodes of the power source pole being respectively electrically connected with the connecting assembly.

## Patentansprüche

1. Zerstäubungsvorrichtung, umfassend: eine äußere Hülse (2), eine Ölspeicherschicht (3), eine Gewebeschicht (4), eine Heizanordnung (6), einen Zerstäubungssitz (7), ein Glasfaserrohr (5) und eine harte Verbindungsanordnung (8), wobei das Glasfaserrohr (5) zum Verpacken durch die Ölspeicherschicht (3) und die Gewebeschicht (4) und zum Tragen der Heizanordnung (6) ausgebildet ist, wobei die Ölspeicherschicht (3), die Heizanordnung (6) und der Zerstäubungssitz (7) in der äußeren Hülse (2) montiert sind; **dadurch gekennzeichnet, dass** der Zerstäubungssitz (7) ein elastischer Zerstäubungssitz ist und einen Verlängerungsabschnitt (72) aufweist, der fest in ein Ende des Glasfaserrohrs (5) eingeführt ist, wobei die harte Verbindungsanordnung (8) in den elastischen Zerstäubungssitz (7) eingeführt ist, um den elastischen Zerstäubungssitz (7) elastisch zusammenzudrücken und spannend mit ihm in Eingriff zu stehen.

2. Zerstäubungsvorrichtung nach Anspruch 1, wobei der elastische Zerstäubungssitz (7) an die äußere Hülse (2) angepasst ist und der elastische Zerstäubungssitz (7) elastisch expandiert wird, wenn die Verbindungsanordnung (8) in den elastischen Zerstäubungssitz (7) eingeführt wird, um in der Innenwand der äußeren Hülse (2) dicht verschlossen zu werden.

3. Zerstäubungsvorrichtung nach Anspruch 1, wobei die Heizeinheit (6) ein Heizelement (60) und elektronische Drähte (61, 63) umfasst, die mit zwei Enden des Heizelements (60) verbunden sind, und die elektronischen Drähte (61, 63) jeweils in dem elastischen Zerstäubungssitz (7) eingespannt und mit der Verbindungseinheit (8) elektrisch verbunden sind.

4. Zerstäubungsvorrichtung nach Anspruch 1, wobei die Verbindungsanordnung (8) einen leitenden Verbinder (81) und eine obere Elektrode (83) aufweist, wobei der leitende Verbinder (81) und die obere Elektrode (83) mit einem zwischen dem leitenden Verbinder (81) und einer oberen Elektrode (83) eingesetzten Isolierring (85) in Eingriff stehen; der elastische Zerstäubungssitz einen Luftkanal (74) und einen Schlitz (73) definiert und der leitende Verbinder (81) spannend in den Schlitz (73) eingesetzt ist; der elastische Zerstäubungssitz (7) einen Luftkanal definiert, und die obere Elektrode (83) in den Luftkanal (74) eingeführt ist, um mit der Innenwand des elastischen Zerstäubungssitzes (7) spannend in Eingriff zu kommen.

5. Zerstäubungsvorrichtung nach Anspruch 4, wobei die Heizeinheit ein Heizelement (60) und elektronische Drähte (61, 63) aufweist, die mit zwei Enden des Heizelements (60) verbunden sind; eine elektronische Leitung (61) zwischen dem elastischen Zerstäubungssitz (7) und dem Verbinder (81) gespannt befestigt ist, um mit dem Verbinder (61) elektrisch verbunden zu werden; die andere elektronische Leitung (63) zwischen der Luftkanalwand des elastischen Zerstäubungssitzes (7) und der oberen Elektrode (83) gespannt befestigt ist, um mit der oberen Elektrode (83) elektrisch verbunden zu werden, und wobei der leitende Verbinder (81) metallisch ist.

6. Zerstäubungsvorrichtung nach Anspruch 5, wobei der elastische Zerstäubungssitz (7) einen Hauptkörper (70) und einen axial verlaufenden Abschnitt (72) aufweist, der in der Mitte des Hauptkörpers (70) ausgebildet ist; der Luftkanal (74) des Zerstäubungssitzes durch den Hauptkörper (70) und den axial verlaufenden Abschnitt (72) verläuft; der Hauptkörper (70) einen ringförmigen Flansch (71) an seiner Außenkante bildet; der Außendurchmesser des ringförmigen Flansches (71) an einen Innendurchmesser der Außenhülse (2) angepasst ist und an der Innenwand der Außenhülse (2) fest verriegelt ist, um eine Dichtwirkung zu erzielen; und wobei der ringförmige Flansch (71) und die Außenwand des Hauptkörpers (70) den Schlitz (73) gemeinsam begrenzen.

7. Zerstäubungsvorrichtung nach Anspruch 5, wobei der elastische Zerstäubungssitz (7) ein Paar von Leitungslöchern (75, 77) bildet, die für die hindurch verlaufenden elektronischen Drähte (61, 63) verwendet werden; die Leitungslöcher (75, 77) jeweils einen dünnen Hals (750, 770) aufweisen, wobei die dünnen Hälse (750, 770) so konfiguriert sind, dass sich der dünne Hals (750, 770) beim Zusammendrücken des Zerstäubungssitzes (7) elastisch verformt, um die elektronischen Drähte (61, 63) darin festzuklemmen.

8. Zerstäubungsvorrichtung nach Anspruch 7, wobei die Leitungslöcher (75, 77) in Längsrichtung durch den Hauptkörper (70) der elastischen Zerstäubungsvorrichtung (7) verlaufen und ein Leitungsloch (75, 77) mit dem Luftkanal (74) in Verbindung steht; eine elektronische Leitung (61) durch ein entsprechendes Leitungsloch (75) verläuft und eine Unterkante des Hauptkörpers (70) umgeht, um sich in den Schlitz (73) zu erstrecken, und durch den Verbinder (81) in der Innenwand des Schlitzes (73) verriegelt ist, und wobei die andere elektronische Leitung (63) durch das andere entsprechende Leitungsloch (77) verläuft, um zur Innenwand des Luftkanals (74) zurück zu führen, und durch die obere Elektrode (83) geklemmt wird.

9. Zerstäubungsvorrichtung nach Anspruch 6, wobei die Außenwand des Verbinders (81) einen ringförmigen Flansch (813) bildet, der an den Innendurchmesser der äußeren Hülse (2) angepasst ist, und der Verbinder (81) darin ein gestuftes Loch bildet; dass wenn der Verbinder (81) in den Schlitz (73) eingeführt wird, der ringförmige Flansch (813) des Verbinders (81) in Längsrichtung dem ringförmigen Flansch (813) des Zerstäubungssitzes (7) widersteht und der Hauptkörper (70) des Zerstäubungssitzes (7) in dem Stufenloch des Verbinders (81) unter Spannung verriegelt ist; die obere Elektrode (83) in dem Stufenloch des Verbinders (81) über einen Isolierring (85) verriegelt ist, sich in Längsrichtung erstreckt und an der Innenwand des Luftkanals (74) des Hauptkörpers (70) des Zerstäubungssitzes (7) unter Spannung verriegelt ist.

10. Zerstäubungsvorrichtung nach Anspruch 1, wobei das Glasfaserrohr (5) ein Montageloch (53) zur Befestigung der Heizeinheit (6) definiert; das Glasfaserrohr (5) ein Doppelschichtrohr mit einem Innenschichtrohr (51) ist; das Innenschichtrohr (51) zur Befestigung der Heizeinheit (6) eine Stützlasche (54) bildet, die dem Montageloch (53) entspricht; und der elastische Zerstäubungssitz (7) aus Silikon besteht.

11. Zerstäubungsvorrichtung nach Anspruch 10, wobei die Ölspeicherschicht (3) eine Lagerwatte ist, die das Glasfaserrohr verpackt.

12. Zerstäubungsvorrichtung nach Anspruch 11, wobei das Glasfaserrohr (5) durch die Gewebeschicht (4) verpackt und anschließend durch die Lagerwatte verpackt ist.

13. Elektronische Zigarette mit einer Zerstäubungsvorrichtung nach einem der Ansprüche 1 bis 12 und einer Batterieanordnung (101), wobei die positiven und negativen Elektroden einer Batterie jeweils mit der Verbindungsanordnung (8) elektrisch verbunden sind.

14. Elektronische Zigarette nach Anspruch 13, wobei die Batterieanordnung (101) in der äußeren Hülse (2) montiert ist.

15. Elektronische Zigarette mit einer Zerstäubungsvorrichtung nach einem der Ansprüche 1 bis 12, und ferner mit einem Stromquellenpol, wobei der Stromquellenpol mit der Zerstäubungsvorrichtung (7) verbunden ist, wobei die positiven und negativen Elektroden des Stromquellenpols jeweils elektrisch mit der Verbindungsanordnung verbunden sind.

## Revendications

1. Dispositif d'atomisation, comprenant : un manchon extérieur (2), une couche (3) de stockage d'huile, une couche (4) de toile, un ensemble chauffant (6), un siège (7) d'atomisation, un tube (5) de fibre de verre et un ensemble (8) de connexion fixe, dans lequel le tube (5) de fibre de verre est configuré pour être garni par la couche (3) de stockage d'huile et la couche (4) de toile et pour supporter l'ensemble chauffant (6), la couche (3) de stockage d'huile, l'ensemble chauffant (6) et le siège (7) d'atomisation étant assemblés dans le manchon extérieur (2) ; **caractérisé en ce que** le siège (7) d'atomisation est un siège d'atomisation élastique et inclut une partie d'extension (72) fermement insérée dans une extrémité du tube (5) de fibre de verre, l'ensemble (8) de connexion fixe est inséré dans le siège (7) d'atomisation élastique pour serrer élastiquement et s'engager en tension avec le siège (7) d'atomisation élastique.

2. Dispositif d'atomisation selon la revendication 1, dans lequel le siège (7) d'atomisation élastique est adapté au manchon extérieur (2), et le siège (7) d'atomisation élastique est élastiquement étendu comme un résultat de l'ensemble (8) de connexion étant inséré dans le siège (7) d'atomisation élastique pour être fermement scellé dans la paroi intérieur du manchon extérieur (2).

3. Dispositif d'atomisation selon la revendication 1, dans lequel l'ensemble chauffant (6) comprend un élément chauffant (60) et des fils électroniques (61, 63) connectés aux deux extrémités de l'élément chauffant (60), et les fils électroniques (61, 63) sont respectivement fixés en tension dans le siège (7) d'atomisation élastique et électriquement connectés à l'ensemble (8) de connexion.

4. Dispositif d'atomisation selon la revendication 1, dans lequel l'ensemble (8) de connexion comprend un connecteur conducteur (81) et une électrode supérieure (83), dans lequel le connecteur conducteur (81) et l'électrode supérieure (83) sont engagés l'un avec l'autre avec une bague d'isolation (85) insérée entre le connecteur conducteur (81) et une électrode supérieure (83) ; le siège d'atomisation élastique définit un canal d'air (74) et une fente (73), et le connecteur conducteur (81) est inséré en tension dans la fente (73) ; le siège (7) d'atomisation élastique définit un canal d'air dans celui-ci, et l'électrode supérieure (83) est insérée dans le canal d'air (74) pour s'engager en tension avec la paroi intérieure du siège (7) d'atomisation élastique.

5. Dispositif d'atomisation selon la revendication 4, dans lequel l'ensemble chauffant comprend un élément chauffant (60) et des fils électroniques (61, 63) connectés aux deux extrémités de l'élément chauffant (60) ; une ligne électronique (61) est fixée en tension entre le siège (7) d'atomisation élastique et le connecteur (81) pour se connecter électriquement au connecteur (81) ; l'autre ligne électronique (63) est fixée en tension entre la paroi de canal d'air du siège (7) d'atomisation élastique et l'électrode supérieure (83) pour se connecter électriquement à l'électrode supérieure (83) et dans lequel le connecteur conducteur (81) est métallique.

6. Dispositif d'atomisation selon la revendication 5, dans lequel le siège (7) d'atomisation élastique comprend un corps principal (70) et une partie (72) s'étendant axialement formée sur le centre du corps principal (70) ; le canal d'air (74) du siège d'atomisation court à travers le corps principal (70) et la partie (72) s'étendant axialement ; le corps principal (70) forme un rebord annulaire (71) sur un bord extérieur de celui-ci ; le diamètre extérieur du rebord annulaire (71) est adapté à un diamètre intérieur du manchon extérieur (2), et verrouille fermement la paroi intérieure du manchon extérieur (2) pour réaliser un effet d'étanchéité ; et le rebord annulaire (71) et la paroi extérieure du corps principal (70) limitent en coopération la fente (73).

7. Dispositif d'atomisation selon la revendication 5, dans lequel le siège (7) d'atomisation élastique forme une paire de trous (75, 77) pour ligne utilisés pour les fils électroniques (61, 63) passant à travers ; les trous (75, 77) pour ligne ont un col mince (750, 770), respectivement, dans lequel les cols minces (750, 770) sont configurés de telle manière que lorsque le siège (7) d'atomisation est serré, le col mince (750, 770) se déforme élastiquement pour pincer fermement les fils électroniques (61, 63) dans celui-ci.

8. Dispositif d'atomisation selon la revendication 7, dans lequel les trous (75, 77) pour ligne passent longitudinalement à travers le corps principal (70) du siège (7) d'atomisation élastique, et un trou (75, 77) pour ligne communique avec le canal d'air (74) ; une ligne électronique (61) passe à travers un trou (75) pour ligne correspondant et contourne un bord de fond du corps principal (70) pour s'étendre dans la fente (73) et est verrouillée dans la paroi intérieure de la fente (73) par le connecteur (81) ; l'autre ligne électronique (63) passe à travers l'autre trou (77) pour ligne correspondant pour revenir dans la paroi intérieure du canal d'air (74) et est verrouillée par l'électrode supérieure (83).

9. Dispositif d'atomisation selon la revendication 6, dans lequel la paroi extérieure du connecteur (81) forme un rebord annulaire (813) adapté au diamètre intérieur du manchon extérieur (2), et le connecteur (81) forme un trou échelonné dans celui-ci ; lorsque le connecteur (81) est inséré dans la fente (73), le rebord annulaire (813) du connecteur (81) résiste longitudinalement au rebord annulaire (813) du siège (7) d'atomisation, et le corps principal (70) du siège (7) d'atomisation est verrouillé en tension dans le trou échelonné du connecteur (81) ; l'électrode supérieure (83) est verrouillée dans le trou échelonné du connecteur (81) par l'intermédiaire d'une bague d'isolation (85), et s'étend longitudinalement et se verrouille en tension dans la paroi intérieure du canal d'air (74) du corps principal (70) du siège (7) d'atomisation.

10. Dispositif d'atomisation selon la revendication 1, dans lequel le tube (5) de fibre de verre définit un trou (53) d'assemblage pour fixer l'ensemble chauffant (6) ; le tube (5) de fibre de verre est un tube à double couche incluant un tube (51) de couche intérieure ; ledit tube (51) de couche intérieure forme une patte support (54) correspondant au trou (53) d'assemblage pour fixer l'ensemble chauffant (6) ; et le siège (7) d'atomisation élastique est constitué de silicone.

11. Dispositif d'atomisation selon la revendication 10, dans lequel la couche (3) de stockage d'huile est du coton de stockage garnissant le tube de fibre de verre.

12. Dispositif d'atomisation selon la revendication 11, dans lequel le tube (5) de fibre de verre est garni par la couche (4) de toile, et ensuite est garni par le coton de stockage.

13. Cigarette électronique, comprenant un dispositif d'atomisation selon l'une des revendications 1 à 12, et comprenant en outre un ensemble (101) de batterie, les électrodes positive et négative d'une batterie étant respectivement électriquement connectées avec l'ensemble (8) de connexion.

14. Cigarette électronique selon la revendication 13, dans laquelle l'ensemble (101) de batterie est assemblé dans le manchon extérieur (2).

15. Cigarette électronique comprenant un dispositif d'atomisation selon l'une quelconque des revendications 1 à 12, et comprenant en outre un pôle de source d'énergie, le pôle de source d'énergie se connectant avec le dispositif (7) d'atomisation, les électrodes positive et négative du pôle de source d'énergie étant respectivement électriquement connectées avec l'ensemble de connexion.
